(19) Europäisches Patentamt European Patent Office Office européen des brevets

(11) **EP 4 782 841 A2**

(12) **EUROPEAN PATENT APPLICATION**

(43) Date of publication:
**29.07.2026 Bulletin 2026/31**

(21) Application number: **26184155.5**

(22) Date of filing: **21.09.2023**

(51) International Patent Classification (IPC):
**G01N 35/04** $^{(2006.01)}$

(52) Cooperative Patent Classification (CPC):
**G01N 35/0099; G01N 35/04;** G01N 2035/0441;
G01N 2035/0446; G01N 2035/0458

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **23.09.2022 IT 202200019581
06.02.2023 EP 23155198**

(62) Document number(s) of the earlier application(s) in
accordance with Art. 76 EPC:
**23776609.2 / 4 591 067**

(71) Applicant: **Diesse Diagnostica Senese S.p.a.
20157 Milano (IT)**

(72) Inventors:
• **BROGI, Alessandra
53035 Monteriggioni (SI) (IT)**

• **BANDINI, Tommaso
53035 Monteriggioni (SI) (IT)**
• **IANNIELLO, Andrea
53035 Monteriggioni (SI) (IT)**
• **MASSARI, Thomas
53035 Monteriggioni (SI) (IT)**
• **RAGANELLI, Luigi
53035 Monteriggioni (SI) (IT)**

(74) Representative: **Botti & Ferrari S.p.A.
Via Cappellini, 11
20124 Milano (IT)**

Remarks:
This application was filed on 10.06.2026 as a
divisional application to the application mentioned
under INID code 62.

(54) **APPARATUS FOR PERFORMING IMMUNOMETRIC TESTS**

(57)     An apparatus (1) for performing immunometric tests is described, comprising a housing element (2) configured to receive and allow tests on at least one test device (D) which is of the single use-type for a single sample test and which includes at least one reaction well (D1) containing a solid phase, a plurality of wells (D2) for reagents, and at least one well (D3) for a sample to be analyzed, a control unit (C) apt to manage the apparatus (1), and at least one image detector (40) in communication with the control unit (C) for acquiring images (Img) of the reaction well (D1). The control unit (C) is configured to process the acquired images (Img) and to perform, on the basis of said processing, a macroarray test relative to the sample in the test device (D), wherein, in said processing, the control unit is configured to identify, at the reaction well (D1) of said test device (D), points of a matrix (M) that includes points of analysis (Pa) related to at least one specific biomarker that represent measurement parameters of said macroarray test, and to perform said macroarray test on the basis of said points. A corresponding method and use procedure is also disclosed.

FIG. 1

**Description**

Field of Application

The present invention refers to an apparatus for performing immunometric tests, in particular using ready-to-use diagnostic devices for a single determination ("monotest or single sample test device"), for example for the identification of autoimmune hepatopathies. The following description refers to this field of application with the only purpose to simplify its exposition.

Prior art

As known, immunometry takes advantage of the antigen-antibody reaction to assess the concentration of a given analyte in a biological sample to be analyzed. To identify a specific antibody, the antigen against which said antibody is directed is used, since, if said antibody is present, it will bind said antigen; alternatively, in a specular way, a specific antibody may be used to detect an antigen. This interchangeability of antigens and antibodies as ligands and as detecting agents underlies the great versatility of the immunometric tests.

The presence of the antigen-antibody complex thus formed, which can be observed by means of specific procedures, is a sign of the presence of the antibody (or of the antigen) that is sought. Thus, immunometric tests allow to detect specific antigens or antibodies in the blood and in other body fluids and are therefore useful for the identification of infectious diseases and other pathological conditions.

As mentioned above, in accordance with known methodologies, the above-mentioned immune reaction is used together with enzymatic reactions to produce a colored signal that can be easily measured in a quantitative way with appropriate detectors.

Nowadays, there are apparatuses capable of performing immunometric tests in a semi-automatic way, providing in a short time quantitative and/or qualitative results.

Among the above-mentioned apparatuses, there are some capable of performing test of the macroarray type in an automatic way, in particular on ninety-six-well plates; in this respect, it should be noted that the use of the above-mentioned ninety-six-well plates limits the applicability of these apparatuses to medium/large analytic routines with the execution of a large number of tests, whereas no flexibility is provided for an analysis on a small number of samples.

The technical problem of the present invention is to provide an apparatus having structural and functional features capable of overcoming the limitations and the drawbacks reported in connection to the prior art, in particular an apparatus that allows to perform tests of the macroarray type in as easy and rapid, and at the same time efficient, way without being limited to the execution of a high number of tests.

Summary of the Invention

The solution idea underlying the present invention is to make an apparatus capable of performing in an automatic way macroarray tests in combination with the use of ready-to-use diagnostic devices for a single determination (namely, the so called monotest or single-sample test devices). In particular, the use of a test device having at least one reaction well for a single sample is provided, said well having a matrix of parameters for the execution of the above-mentioned macroarray test by processing of images of said well.

On the basis of said solution idea, the above-mentioned technical problem is solved by an apparatus for performing immunometric tests comprising a housing element configured to receive and allow tests on at least one test device which is of the single use-type for a single sample test and which includes at least one reaction well containing a solid phase, a plurality of wells for reagents, and at least one well for a sample to be analyzed, a control unit apt to manage the apparatus, and at least one image detector in communication with the control unit for acquiring images of the reaction well, wherein the control unit is configured to process the acquired images and to perform, on the basis of said processing, a macroarray test relative to the sample in the test device, and wherein, in said processing, the control unit is configured to recognize (identify), at the reaction well of said test device, points or elements (also called "spots") of a matrix; among said points or elements of the matrix there are points of analysis (that are thus arranged as in a matrix) related to at least one specific biomarker (and also possibly to a plurality thereof), that represent measurement parameters of said macroarray test, and to perform said macroarray test on the basis of said points.

The test device may be considered as part of the apparatus, which therefore comprises at least one of said devices.

More particularly, the invention comprises the following additional and optional features, taken individually or in combination if necessary.

According to an aspect of the present invention, the matrix may comprise also a plurality of reference points in a fixed position, said reference points being arranged to allow to recognize the orientation of the points of analysis of the matrix within the acquired image, and wherein the control unit is configured to perform a procedure of virtual alignment of the matrix based on said reference points.

According to an aspect of the present invention, the control unit may be configured to identify and define the points of the matrix by implementation of a calculation procedure based on Hough transform.

According to an aspect of the present invention, wherein the control unit may be configured to:

- define, for each image and for each of the identified points of analysis, a pixel array including the pixels included in said single points of analysis; and

- define also, for each image, a pixel array including the pixels not included in said identified points of analysis.

According to an aspect of the present invention, the control unit may be further configured to calculate the average value of the pixels in said pixel arrays and to calculate, on the basis of said average values, the intensity of each one of the identified points of analysis.

According to an aspect of the present invention, the control unit may be configured to perform a calibration procedure based on one or more known calibration points within the matrix that represent the dynamic range of the measurement parameters, said calibration points being defined within a calibration well specifically apt to perform said calibration procedure, or within the matrix of the reaction well itself.

According to an aspect of the present invention, the matrix may comprise at least one 6x6 grid or one 8x8 grid for the points of analysis, and wherein each measurement parameter is tested in duplicate or triplicate within the reaction well.

According to an aspect of the present invention, the test device may comprise a body which houses, along its longitudinal axis, the reaction well containing a solid phase, the plurality of wells for reagents, and the well for the sample to be analyzed, and wherein said test device comprises, along said longitudinal axis, at least one recess to receive the reaction well from a preexisting microtitration plate, wherein the solid phase comprises purified native proteins or recombinant proteins that are printed on the bottom of said microtitration plate, which is subdivided into said reaction wells which are individually inserted into said test device, and wherein the matrix is formed by means of a coating of said reaction well.

According to an aspect of the present invention, the apparatus may comprise one or more needles controlled in an automatic way by the control unit for transferring the samples and reagents for carrying out the immunometric test, and wherein said control unit is configured to control said needles so as to perform the following analysis steps:

- addition of the sample to be analyzed into the reaction well and incubation of the same in said reaction well;

- washing of the reaction well;

- addition of a conjugated tracer into the reaction well and incubation of the same in said reaction well;

- further washing of the reaction well; and

- addition of a macroarray substrate into the reaction well.

According to an aspect of the present invention, the housing element may be configured to house a plurality of said test devices, said apparatus further comprising means apt to act on the housing element to allow the execution of the immunometric test.

According to an aspect of the present invention, said biomarkers may be related to an autoimmune disease.

According to an aspect of the present invention, said biomarkers may comprise the antinuclear autoantibody (ANA), the anti-smooth muscle autoantibody (ASMA) and the anti-soluble liver antigen autoantibody (anti-SLA), said autoimmune disease being type-1 autoimmune hepatopathy (EAI-1).

According to an aspect of the present invention, said biomarkers may comprise: the anti-liver-kidney microsomal autoantibody (anti-LKM1), and the anti-liver cytosolic antigen type-1 autoantibody (anti-LC1), said autoimmune disease being type-2 autoimmune hepatopathy (EAI-2).

According to an aspect of the present invention, said biomarkers may comprise: the anti-mitochondrial autoantibody (AMA), the anti-M2 autoantibody (anti-M2), the anti-F-actin antibody (anti-F-actin), the sp100 antibody (anti-sp100) and the gp210 antibody (anti-gp210), said autoimmune disease being primary biliary cirrhosis (PBC).

According to an aspect of the present invention, said matrix (M) may include points of analysis related to at least two biomarkers, preferably at least three biomarkers, more preferably at least four biomarkers, even more preferably at least five biomarkers, conveniently six biomarkers selected from said biomarkers of EAI-1, EAI-2 and/or PBC, said six biomarkers preferably being the anti-mitochondrial autoantibody (AMA), the sp100 antibody (anti-sp100), the gp210 antibody (anti-gp210), the anti-liver-kidney microsomal autoantibody (anti-LKM1), the anti-liver cytosolic antigen type-1 autoantibody (anti-LC1), and the anti-soluble liver antigen autoantibody (anti-SLA).

The present invention also refers to a corresponding method for performing immunometric tests.

In particular, the method comprises:

- arranging a housing element configured to receive and allow tests on at least one test device which is of the single use-type for a single determination (single sample test), and which includes at least one reaction well containing a solid phase, a plurality of wells for reagents, and at least one well for a sample to be analyzed;

- acquiring images of the reaction well;

- processing the images acquired;

- performing, on the basis of said processing, a macroarray test relative to the sample in the test device, wherein said processing comprises recognizing (identifying), at the reaction well of said test device, points or elements (also called "spots") of a matrix; among said points or elements of the matrix there are points of analysis related to at least one specific biomarker (and also possibly to a plurality thereof), that represent measurement parameters of said macroarray test; and

- performing said macroarray test based on said points.

According to an aspect of the method of the present invention, the matrix may comprise at least one 6x6 grid or one 8x8 grid for the points of analysis, and wherein each measurement parameter is tested in duplicate or triplicate within the reaction well.

According to an aspect of the method of the present invention, said biomarkers may be related to an autoimmune disease.

According to an aspect of the method of the present invention, the method may comprise the step of selecting the biomarkers from the antinuclear autoantibody (ANA), the anti-smooth muscle autoantibody (ASMA) and the anti-soluble liver antigen autoantibody (anti-SLA), said autoimmune disease being type-1 autoimmune hepatopathy (EAI-1).

According to an aspect of the method of the present invention, the method may comprise the step of selecting the biomarkers from: the anti-liver-kidney microsomal autoantibody (anti-LKM1), and the anti-liver cytosolic antigen type-1 autoantibody (anti-LC1), said autoimmune disease being type-2 autoimmune hepatopathy (EAI-2).

According to an aspect of the method of the present invention, the method may comprise the step of selecting the biomarkers from: the anti-mitochondrial autoantibody (AMA), the anti-M2 autoantibody (anti-M2), the anti-F-actin antibody (anti-F-actin), the sp100 antibody (anti-sp100) and the gp210 antibody (anti-gp210), said autoimmune disease being primary biliary cirrhosis (PBC).

According to an aspect of the method of the present invention, said matrix may include points of analysis related to at least two biomarkers, preferably at least three biomarkers, more preferably at least four biomarkers, even more preferably at least five biomarkers, conveniently six biomarkers selected from said biomarkers of EAI-1, EAI-2 and/or PBC, said six biomarkers preferably being the anti-mitochondrial autoantibody (AMA), the sp100 antibody (anti-sp100), the gp210 antibody (anti-gp210), the anti-liver-kidney microsomal autoantibody (anti-LKM1), the anti-liver cytosolic antigen type-1 autoantibody (anti-LC1), and the anti-soluble liver antigen autoantibody (anti-SLA).

The invention also refers to the use of the above apparatus for the detection of autoimmune diseases.

For example, in this application the biomarkers may be selected from:

- the antinuclear autoantibody (ANA), the anti-smooth muscle autoantibody (ASMA) and the anti-soluble liver antigen autoantibody (anti-SLA), said autoimmune disease being type-1 autoimmune hepatopathy (EAI-1); and /or

- the anti-liver-kidney microsomal autoantibody (anti-LKM1), and the anti-liver cytosolic antigen type-1 autoantibody (anti-LC1), said autoimmune disease being type-2 autoimmune hepatopathy (EAI-2); and/or

- the anti-mitochondrial autoantibody (AMA), the anti-M2 autoantibody (anti-M2), the anti-F-actin antibody (anti-F-actin), the sp100 antibody (anti-sp100) and the gp210 antibody (anti-gp210), said autoimmune disease being primary biliary cirrhosis (PBC).

The features and advantages of the apparatus and the method according to the invention will become apparent from the following description of an embodiment thereof, given by way of non-limiting example with reference to the accompanying drawings.

Brief description of the figures

In the figures:

- Figure 1 is a general outline of the apparatus according to the present invention;

- Figure 2 shows an example of a test device used by the apparatus according to the present invention;

- Figure 3 is a schematic representation of steps of a macroarray test performed according to the present invention;

- Figures 4A and 4B show examples of a matrix of points that is formed in a reaction well in accordance with embodiments of the present invention;

- Figure 5 is an example of an image of a reaction well wherein a matrix of points is visible;

- Figure 6 shows an example of a procedure of alignment in an image according to embodiments of the present invention;

- Figure 7 shows an example of a grayscale image wherein an intensity value is assigned to each pixel;

- Figure 8 shows the result of the analysis on the matrix of Figures 5 and 6;

- Figures 9A-9D show examples of calibration layouts in accordance with embodiments of the present invention;

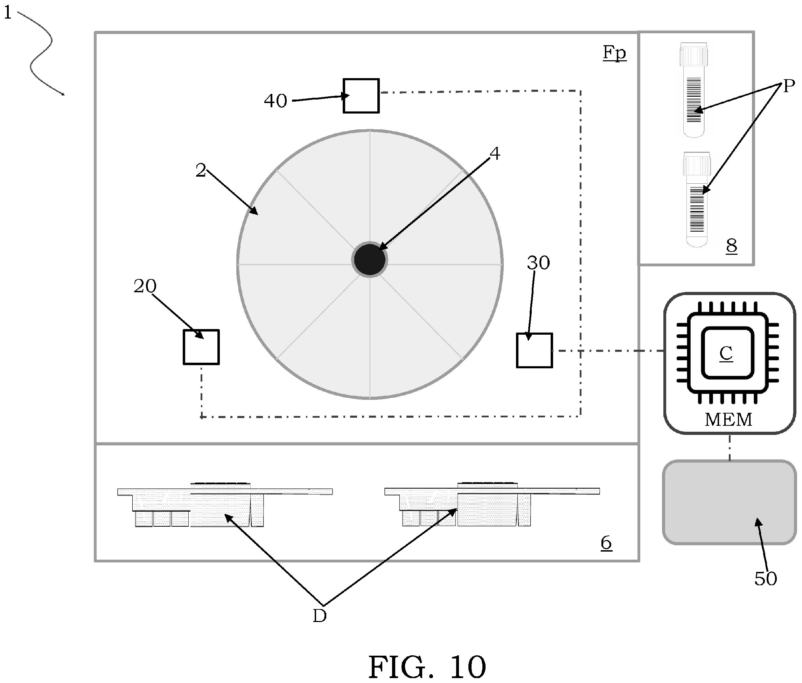

- Figure 10 is another general outline of an apparatus according to an embodiment not part of the present invention, that comprises additional components;

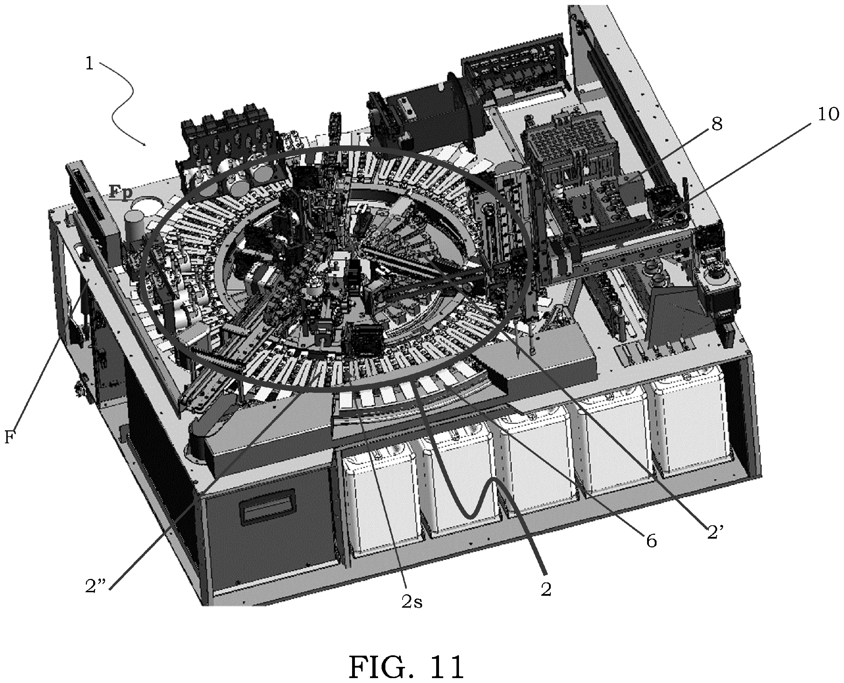

- Figure 11 is a perspective view of the apparatus according to embodiments not part of the present invention;

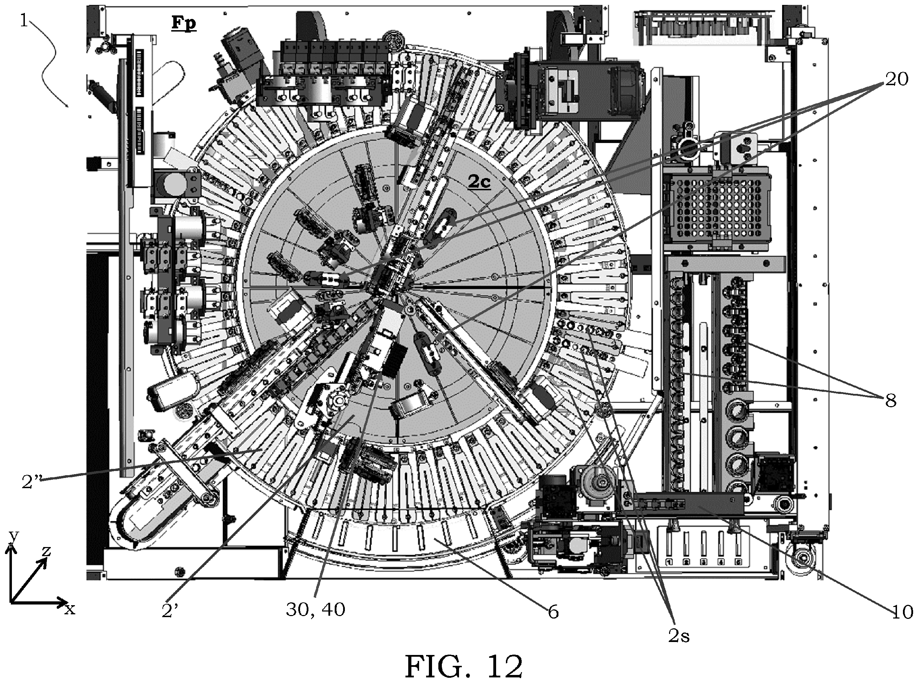

- Figure 12 is a top view of the apparatus of Figure 11 not part of the present invention;

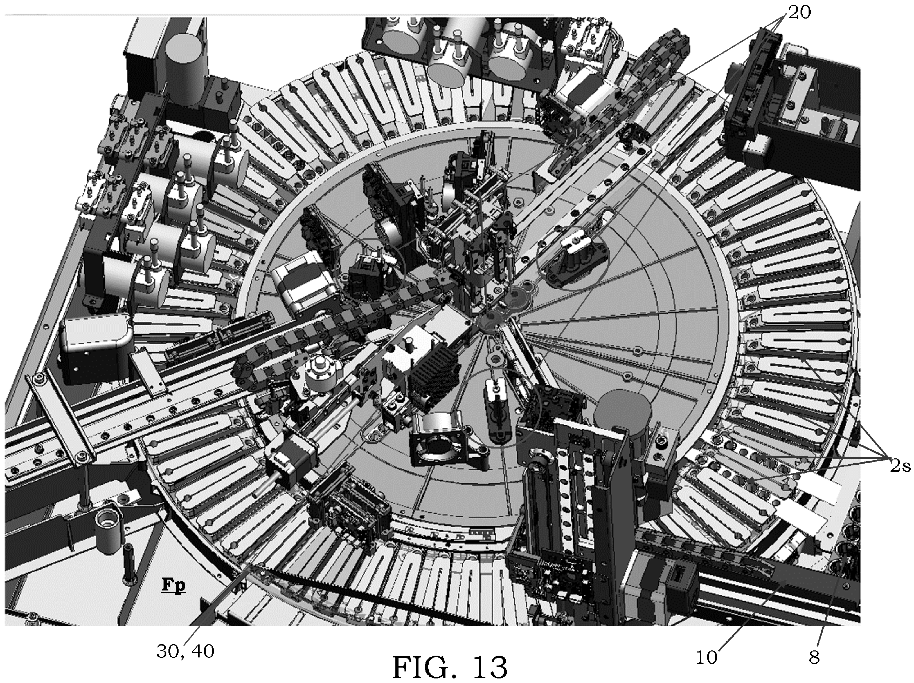

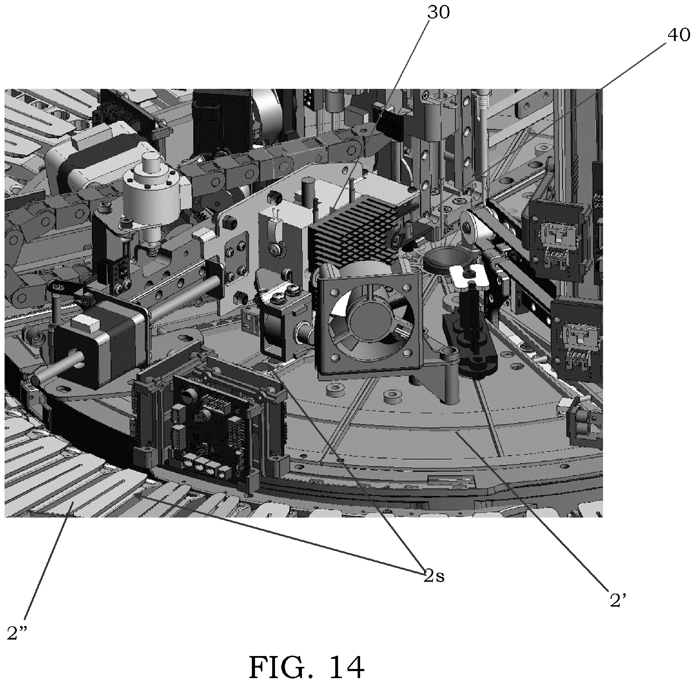

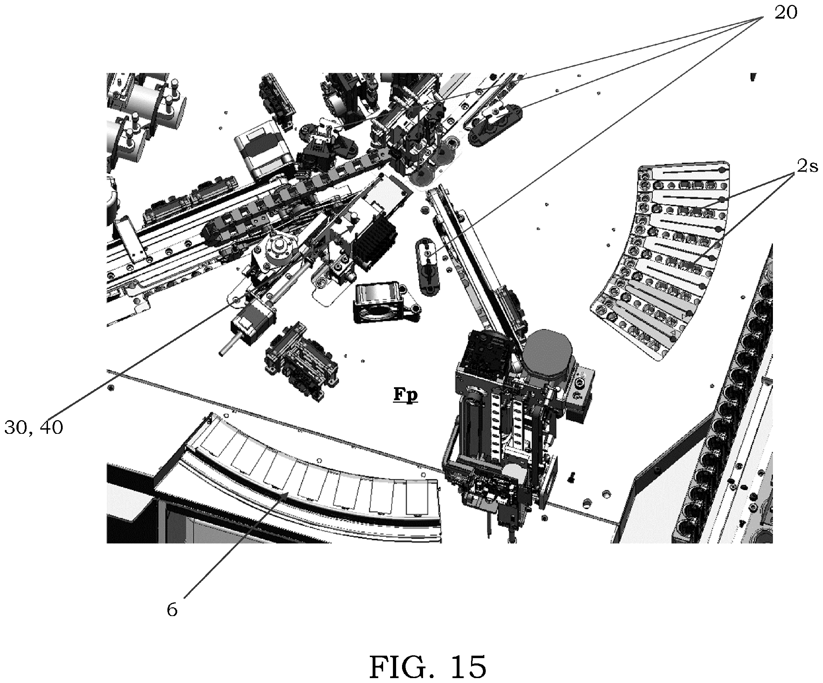

- Figures 13, 14 and 15 show details of the apparatus in accordance with embodiments not part of the present invention; and

- Figures 16A and 16B show examples of an application of the apparatus 1 to the analysis of autoimmune hepatopathies.

Detailed Description

With reference to those figures, an apparatus for performing immunometric tests according to the present invention is globally and schematically indicated with the reference number 1.

It is worth noting that the figures represent schematic views and are not necessarily drawn to scale, but instead they are drawn so as to emphasize the important features of the invention. Further, in the figures, the different elements are showed in a schematic way, their shape being variable depending on the desired application. It is further worth noting that in the figures identical reference numerals refer to identical elements in shape or function. Finally, particular features described in relation to an embodiment illustrated in a figure are also applicable to other embodiments illustrated in the other figures.

It is also noted that, unless the opposite is expressly indicated, the described process steps may also be inverted if necessary.

The present description shows an apparatus 1 for performing macroarray tests, said apparatus 1 being configured to receive and use specific diagnostic devices (hereinafter also called "test devices" and being indicated with reference D), which are ready-to-use and for a single determination (i.e. they are monotest devices).

For the purpose of allowing the execution of the operations described below, the apparatus 1 comprises a control unit (identified with reference C), including appropriate memory units MEM and suitably programmed and designated for managing and automatically controlling the apparatus and for the analysis of the data of measurement. The control unit C may be, for example, a computerized unit integrated in the apparatus 1. Moreover, it is noted that the control unit C may be a single unit or may comprise a plurality of local and/or remote units, possibly communicating with each other and each being designated for performing specific operations. The control unit C is thus capable of controlling the apparatus 1 to obtain the desired functionalities. In any case, the present invention is not, in any way, limited by the architecture used for the control unit C, which may be in general any suitable computerized unit, comprising one or more unit(s) depending on the needs and/or circumstances.

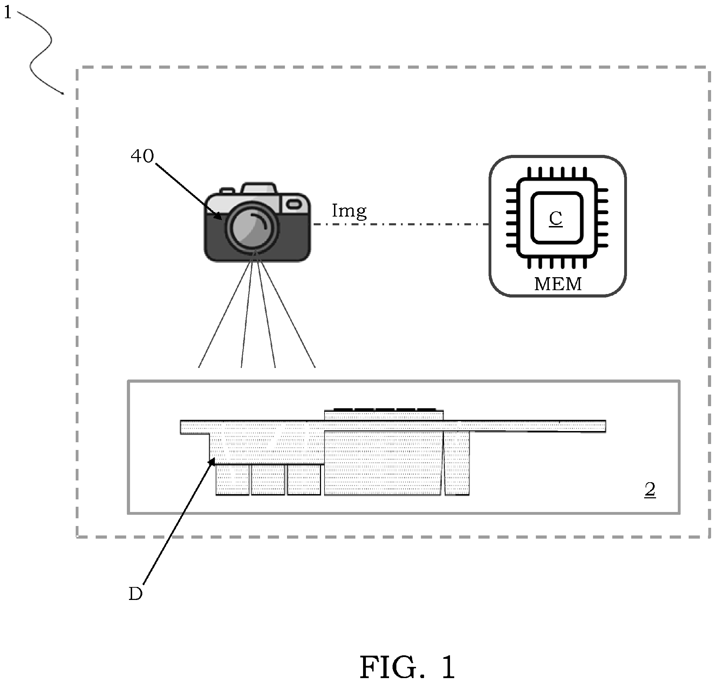

As illustrated in Figure 1, in its most general form, the apparatus 1 is structured and configured to function with at least one test device D which is of the single use-type for a single determination. In particular, these test devices D can be inserted in appropriate housing seats of a housing element (indicated with the reference number 2) for the execution of the desired test and can be extracted at the end of the test, based on a mechanical structure that will be illustrated in more detail hereinafter.

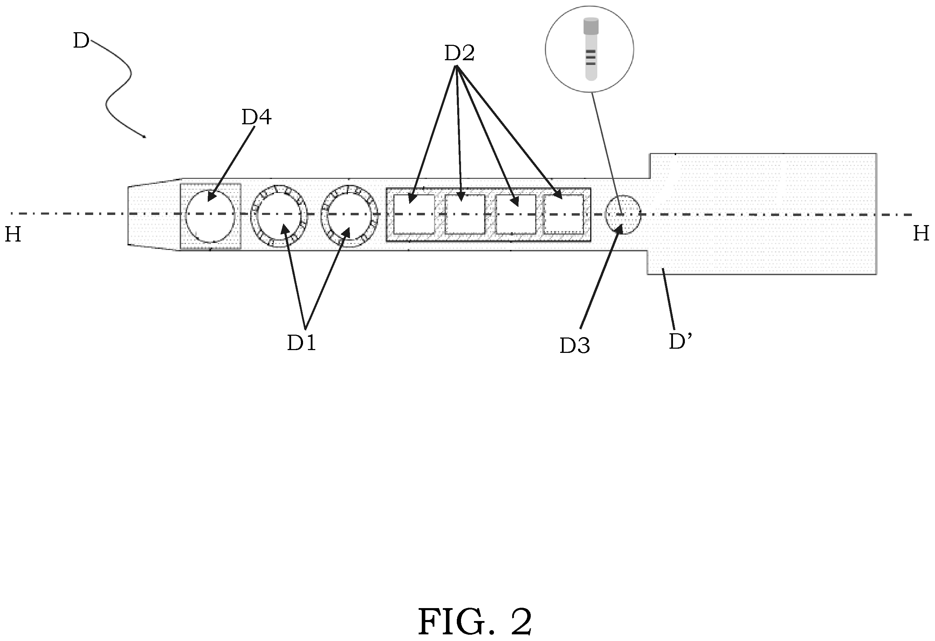

With reference to Figure 2, the test device D comprises at least one reaction well D1 containing a solid phase, a plurality of wells D2 for reagents, and at least one well D3 for a sample to be analyzed.

As it will be disclosed in the following, for example, the solid phase may comprise purified native proteins or recombinant proteins that are printed on the bottom of a microtitration plate, which is subdivided into the single reaction wells which are individually inserted into said test device D.

In other words, generally, the test device D comprises, along the longitudinal axis thereof, at least one recess to receive the reaction well from a preexisting microtitration plate, i.e. to receive the so-called solid phase; for this reason, in the context of the present disclosure, the solid phase is the bottom of the reaction well, which comes from the subdivision of the existing microtitration plate. In accordance with embodiments of the present invention, the solid phase of the well thus corresponds to the bottom of the well to which a coating of biomarker-specific antigen protein is applied.

This is very advantageous since this simple technique allows to perform one or more tests on a single sample without being bound to a plate with a plurality of wells, for example ninety-six wells, as in the prior art solutions.

In this way, a very compact test device may be used, in order to provide a simple yet effective apparatus, for a very fast and effective determination, which is very useful especially for the execution of macroarray test.

The apparatus 1 further comprises a reading unit comprising at least one image detector 40 in communication with the control unit C for acquiring images (indicated with the reference Img) of the reaction well D1.

In an embodiment, the image detector 40 is a camera having high resolution (for example, from 5 MP to 15 MP) and capable of adjusting the focus, the exposure and other parameters; the resolution of the images may be, for example, 1920×1080, or it may be equal to even higher values, such as for example 2592×1944, but it is obviously not limited to said values. The images Img may be saved in .png format in order to possibly allow their compression without loss of information.

The control unit C is configured to process the acquired images Img and to perform, based on said processing and based on instructions stored therein (in particular, based on an image processing algorithm), a macroarray test relative to the sample in the test device D.

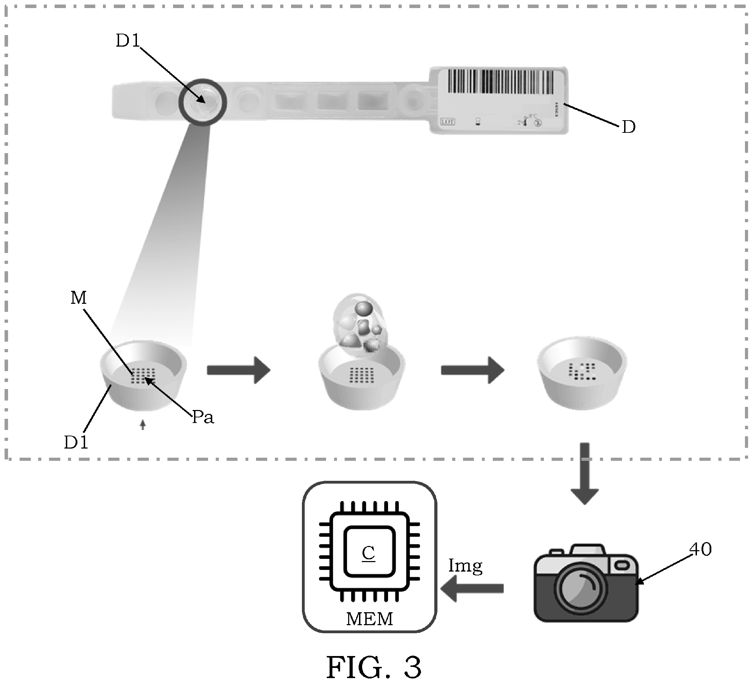

In particular, as schematically shown in Figure 3, the reaction well D1 of the test device D comprises a coating, which has a matrix or grid M that includes points of analysis (identified with the reference Pa, also called "Spots") which are related to specific biomarkers representing the measurement parameters of the macroarray test.

The measurement cycle comprises the introduction of the diluted sample (for example, 1:100) into the reaction well D1, in which it is subjected to an incubation process for about twenty minutes, at the end of which, washing of said reaction well D1 is performed. Conjugated antibodies (for example, conjugated with peroxidase) are then added into the reaction well D1, after which an additional incubation and subsequent washing follow. The substrate for making the points of matrix M on the bottom of the reaction well D1 visible is then supplied, and everything ends with a last washing. Finally, the image detector 40 acquires the image Img and the desired result is provided by processing said images Img.

As it will be also shown hereinafter, in order to perform said measurement cycle, the apparatus 1 comprises one or more needles that are controlled in an automatic way by the control unit C and act on the test device D for transferring the samples and reagents. Summing up, the control unit C is thus configured to control said needles so as to perform the following analysis steps:

- addition of the sample to be analyzed into the reaction well D1 and incubation of the same in the reaction well D1;

- washing of the reaction well D1;

- addition of a conjugated antibody into the reaction well D1 and incubation of the same in said reaction well D1;

- further washing of the reaction well D1;

- addition of a macroarray substrate into the reaction well D1;

- last washing; and

- acquisition of one or more images Img.

The expression "macroarray substrate" herein indicates the reagent required for analyte detection, i.e. the last reactant that is added to the reaction to cause the formation of the colored precipitate in the spot (that is what is detected with the analysis of the image from the camera). Specifically, it is a reactant that is oxidized by the enzyme peroxidase present in the conjugate; preferably, the reactant is tetramethylbenzidine; in particular, tetramethylbenzidine precipitates and forms the color within the spot.

In an embodiment, the control unit C is configured to process the acquired images Img by means of machine learning techniques.

In an embodiment, the image detector 40 is capable of moving in one direction only (for example a direction parallel to the working plane on which the apparatus 1 lies), even if other configurations are possible and fall within the scope of the present invention.

The camera 40 is preferably configured to acquire a plurality of images (without limitation to a specific number) that will be processed later.

Further, in order to illuminate the spots lying on the bottom of the reaction well D1 of the test device D without generating light reflections, the macroarray reading unit comprises also an appropriate illumination system (not shown in the figures) which is suitably calibrated.

Conveniently, the macroarray allows the determination of several analytes in a single test, with a single blood draw, wherein the reaction well D1 comprises the above-mentioned matrix capable of making specific spots corresponding to specific reactions appear. Thus, the present invention implements a method of detection by images in combination with a package of automated data analysis, thereby obtaining multi-parameter results within the same reaction well, with small sample volumes, thanks to the above-mentioned acquisition of the image of the reaction well (in particular of its grid) after the reaction has occurred, with the appearance of specific spots that are sensitive to respective reacting samples.

According to embodiments of the present invention, therefore, the above well is a microwell that has been sensitized with specific biomarkers (proteins/antibodies that are specific for the diagnosis of the relative disease). In particular, the biomarkers are antigenic proteins related to the disease, which are adhered to specific points of the well (Microspots) and are arranged in specific points of the reference matrix.

As used herein, the term "macroarray" is equivalent to "Microspot ELISA", and refers to a sensitized microwell as described above, made by "non-contact printer" instruments, capable of depositing a few tenths (for example, 0.2/0.4 μL) of solutions containing the selected antigens, with appropriate precision dispensers and following a precise layout according to the present invention.

In the classic microwells that have been sensitized for the execution of an ELISA test, instead, the antigen is adhered to the microplate well by dispensing volumes that indiscriminately cover the entire surface of the well bottom.

In accordance with embodiments of the present invention, the solid phase of the well comprises the bottom of the well to which a coating of biomarker-specific antigen protein arranged as a matrix is applied.

Preferably, the plastic which the well is made of is white polystyrene. More preferably, a white HB Biomat plate with high binding ability is used, so that no covalent bonds, but rather adhesions by electrostatic interactions such as, e.g., weak van-der-Waals bonds, are formed.

In another, equally preferred, embodiment, the spot may be also performed on MB (with medium binding ability) polystyrene plastics, or on NB (no binding ability) polystyrene plastics.

In another alternative, equally preferred, embodiment, the spot may be performed on plastics pre-functionalized for the covalent bond on COOH or on NH2 or on the sulfur atoms of cysteine residues, or on plates with poly-L-Lysine or poly-L-Arginine.

In the examples shown below in the present description, a diagnostic methodology based on the measurement of six parameters (in particular for the identification of autoimmune hepatopathies, as it will be detailed hereinafter) will be explained, even if the present invention is not limited thereto and many other applications are possible.

Referring again to Figure 2, the test device D comprises a body D' which houses, along its longitudinal axis H-H, the above-mentioned reaction well D1 containing a solid phase (possibly, more than one reaction wells may be provided), the above-mentioned plurality of wells D2 for reagents, and the well D3 for the sample to be analyzed.

More in particular, the test device D comprises, along its longitudinal axis H-H, at least one recess to receive the reaction well D1, which comes from a preexisting microtitration plate, for example a ninety-six-well plate. The solid phase may comprise biomarkers, for example purified native proteins or recombinant proteins, printed on the bottom of the microtitration plate, which is subdivided into many reaction wells D1 which, once separated from the plate, are individually inserted into the test device D. This is very advantageous since this simple technique allows to perform one or more tests on a single sample without being bound to a plate with a plurality of wells, for example ninety-six wells.

The test devices D are thus configured to be of the single use-type and the apparatus 1 is configured to receive said test devices D. In the example shown, there are two wells D1 so as to be able to perform two determinations on the same sample. In other words, in a preferred embodiment, in order to improve the precision of the test, a second sensitized reaction well D1, with the same structure as the first, is inserted in each test device D, thus making a technical replicate in the same monotest.

Referring again to Figure 2, there is then possibly an end recess D4 for blank execution (only substrate).

The material of the test device D is not limited to a particular type and may be, for example, any plastic material suitable for the purpose.

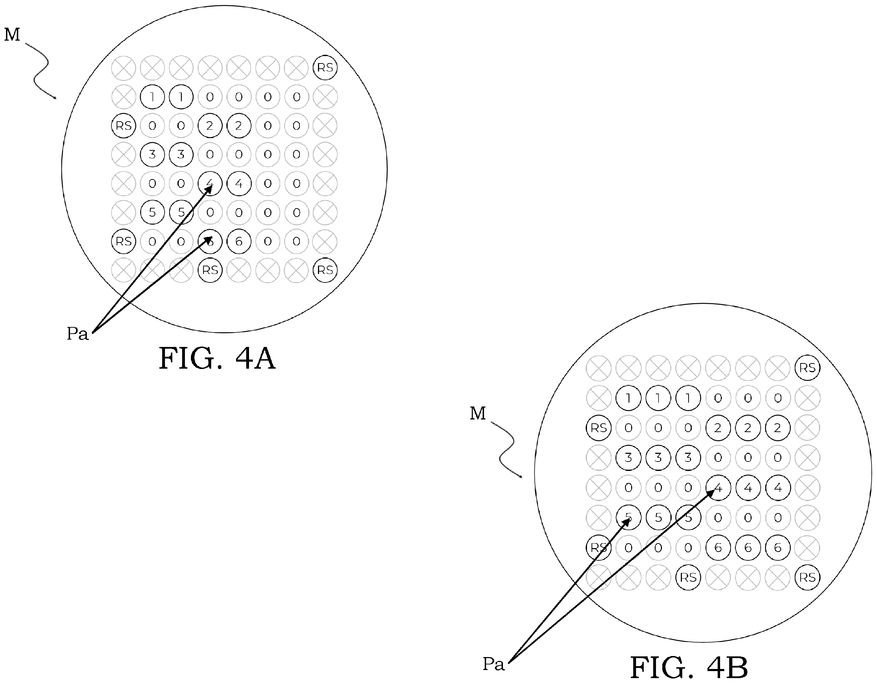

Figures 4A and 4B show examples of the matrix M formed on the reaction well D1 for the execution of the macroarray test according to embodiments of the present invention.

In particular, Figure 4A shows the layout of matrix M for an analysis in duplicate, whereas Figure 4B shows the layout of matrix M for an analysis in triplicate. In fact, as seen above, each single parameter may be tested in duplicate or in triplicate within the same reaction well and the numerical value of intensity of pixels obtained by processing the image Img is the result of the average of the two or three values obtained. In any case, Figures 4A and 4B are only exemplary and many other layouts are possible for matrix M, as will be also illustrated hereinafter in this description.

In an exemplary embodiment that will be illustrated in more detail hereinafter, six measurement parameters are considered and the points of analysis Pa form thus a 6x6 grid, although in various applications the grid may also be a 8x8 grid, or may have an even different size.

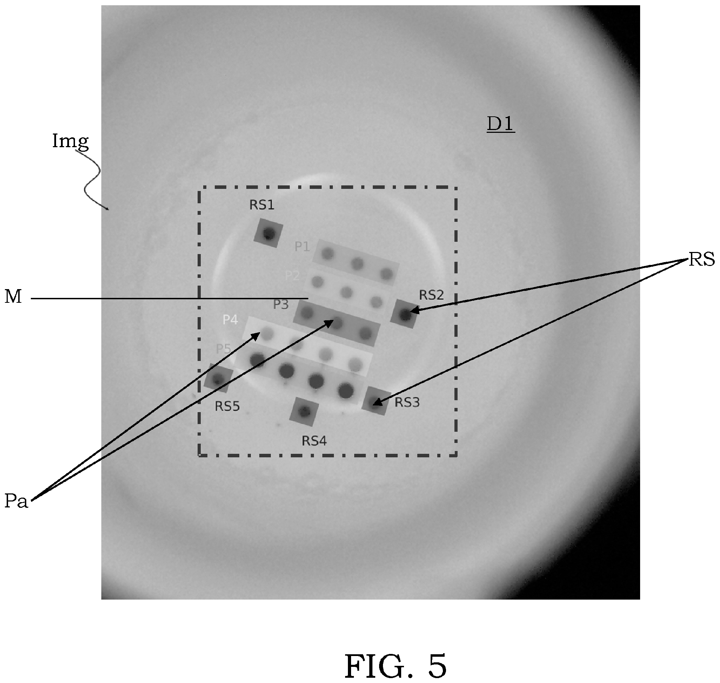

Figure 5 shows an example image Img of the reaction well D1, in which it is possible to see the points of analysis Pa that appear at the end of a reaction (in the Figure called P1-P5), which may obviously vary from test to test (the image shown is in fact only an example aimed to generally describe the present invention, and many other solutions are possible).

Figures 5, 6 and 8 describe a particular example in which the matrix is a 6x6 matrix. In other applications, as mentioned above and as will be illustrated in the following figures, other matrices, such as for example 8x8 matrices, may be used.

As mentioned, generally, the matrix composing the panel of detection of specific biomarkers (for example, but not limited to, for the analysis of autoimmune hepatopathies) may be a 6x6 matrix, a 8x8 matrix (sixty-four positions), but with the possibility of further expansion (for example, 10x10, 15x15).

As illustrated in the figures, the coating of the reaction well D1 comprises also a plurality of reference points (indicated with the reference RS) included in the matrix M in a fixed position. These reference points RS are arranged to orient the image with respect to a known and fixed reference and thus to recognize without mistakes which of the spots is associated to a given analyte (biomarker).

More in particular, the above-mentioned reference points RS are arranged to allow to define the orientation of matrix M within the acquired image Img, and the control unit C is configured to perform a procedure of virtual alignment of matrix M on the basis of said reference points RS.

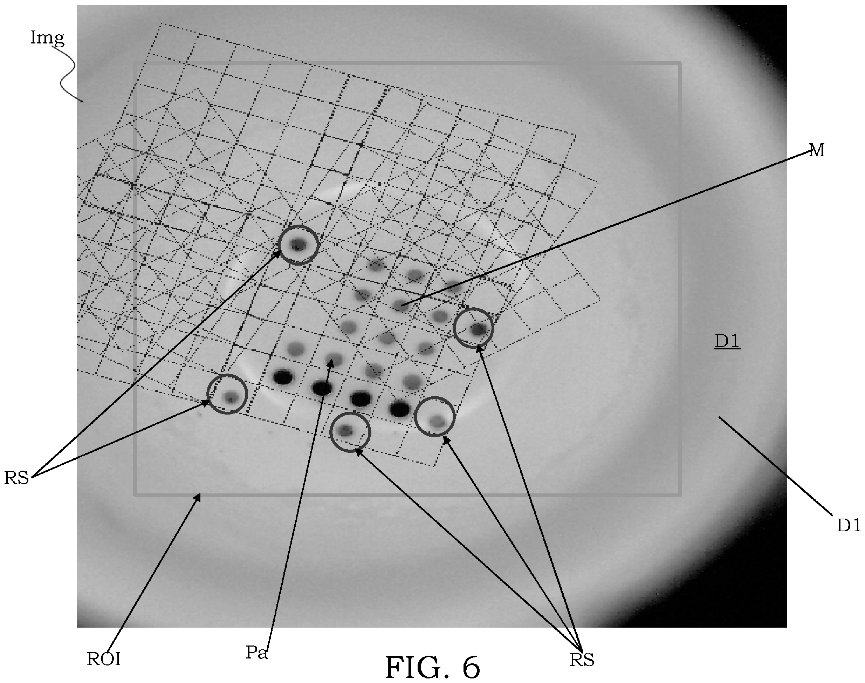

Even more in particular, in said alignment procedure, the control unit C performs a series of calculations with the purpose of evaluating the orientation of the reaction well D1 within the image Img acquired on the basis of the reference points RS, and thus identifying which points of analysis are visible and where they are positioned. As shown in the example of Figure 6, firstly, an area of analysis or ROI ("Region Of Interest") is defined (for example, but not limited to, by defining a square or a rectangle), wherein everything that is outside of said area is not considered, thus speeding up the analysis. A Gaussian filter is then applied in order to perform a smoothing operation on the image Img and to reduce the noise as much as possible. Once this has been done, the points of matrix M are identified by Hough transform, with particular reference to the reference points RS. The relative distances of the centers of the various points are then calculated, and the points with the largest distances are the reference points RS, since said points are at the far end of the grid. Finally, the angle of inclination between two of said reference points RS is calculated, said angle thus providing the orientation of matrix M in the image Img. The matrix M is finally rotated until the reference points RS are in reference positions of a stored reference grid. In this way, the image is oriented with respect to a preset reference system in order to perform the analysis always under the same conditions.

In the shown example, the reference points RS are five, but any suitable number may be used.

It is further noted that the first steps of this procedure carried out by the central unit C, namely the definition of an area of analysis, the application of a filter (for example a Gaussian filter), and the recognition of the points of matrix M by implementation of a calculation procedure based on Hough transform, are a general feature of the present invention for recognizing and defining the points of analysis that appear in the acquired Img.

Once the matrix M has been identified and it has been preliminarily assessed where the points of analysis are (in accordance with the above-described procedure), a more accurate analysis of the single points is then made, in order to extract parameters useful for the desired test.

In particular, by means of the central unit C an analysis is carried out in which, in order to define the contour of the various spots, the Hough transform is performed again, iterating the parameters until the circumference delimiting said spots is determined. The radius and the center of this circumference are then calculated; this is iterated in the whole area of analysis in order to distinguish the pixels within the points of analysis Pa and those outside said points of analysis Pa (i.e. the background), thus defining different areas of pixels.

In other words, in an embodiment of the present invention, the control unit C is configured to define, for each of the recognized points of analysis Pa, a pixel array including the pixels relative to said single points of analysis Pa (i.e. the pixels included in said points of analysis), and also to define, for each image Img, a pixel array including the pixels not included in the points of analysis Pa.

In this embodiment, the control unit C is further configured to calculate the average value of the pixels in the various identified arrays, and then to calculate, based on said average values, the intensity of each of the recognized points of analysis Pa (for example, as the difference between average background and average of a point).

In general, there are n spot arrays as many are the n spots and a single background array.

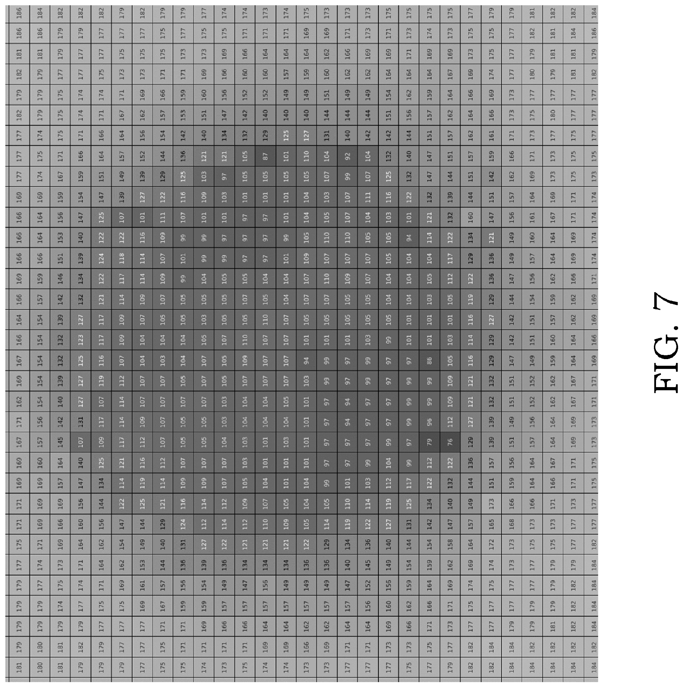

In an embodiment, the image Img is acquired in RGB colors, but is subsequently converted into a grayscale for analysis, wherein an intensity from 0 to 255 is assigned to each pixel, see for example Figure 7.

Summing up, for each point of analysis Pa, two arrays are considered, one containing the position of the pixels within the point of analysis and the other one containing the position of the pixels outside the points of analysis (this array being the same for all the points), and for each of these arrays the average intensity is calculated. The difference between the averages gives therefore the intensity value of the point. As mentioned above, since each parameter is tested in duplicate or in triplicate, the intensity of each parameter is given by the average of the intensity of the points relative to that parameter (two or three points), according to the formula:

$$Intesity_{P1} = \frac{1}{n} \sum_{i=1}^{n} Intensity_i$$

If the intensity of a parameter is above a specific threshold value, the sample is considered reactive for that specific parameter; in particular, an intensity index (whose calculation will be detailed below) is assigned to each parameter and said intensity index is compared to a reference threshold value.

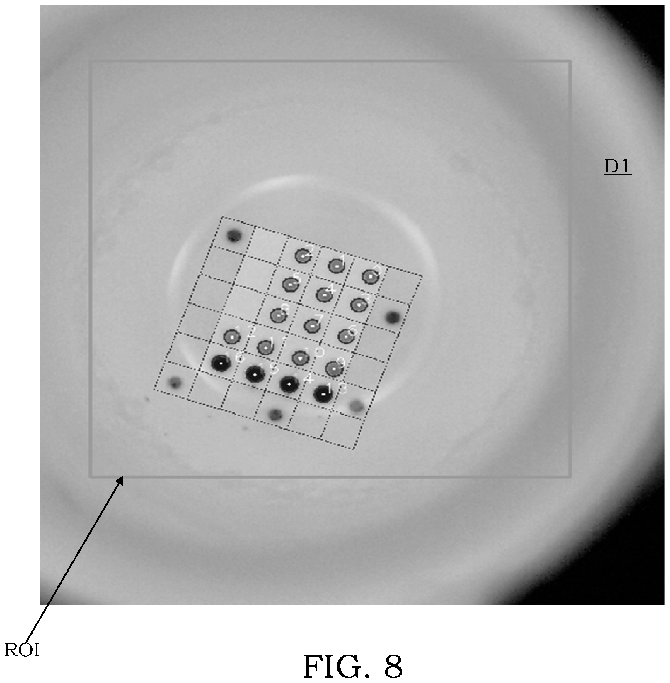

Figure 8 is an example of an image of the reaction well D1, wherein a single pixel corresponds to 15 $\mu$m with an image resolution of 1920x1080. On average, each point corresponds to twenty-four pixels with an area of 0.102 mm$^2$.

Other embodiments are also possible, wherein an algorithm for the recognition of regions (known in the art as "Blob Detection Algorithm") is performed, with which it is possible to define the contours on the basis of pixels intensity variations.

In order to perform an accurate analysis, the control unit C is further configured to perform a calibration procedure based on one or more known calibration points (identified with the reference Cal) within the matrix M that represent the dynamic range of the measurement parameters. These calibration points Cal may be defined within a calibration well specifically apt to perform said calibration procedure, or within the matrix of the reaction well D1 with which the test is carried out.

In an embodiment, the calibration procedure is performed on at least five points representing the dynamic range of the various parameters.

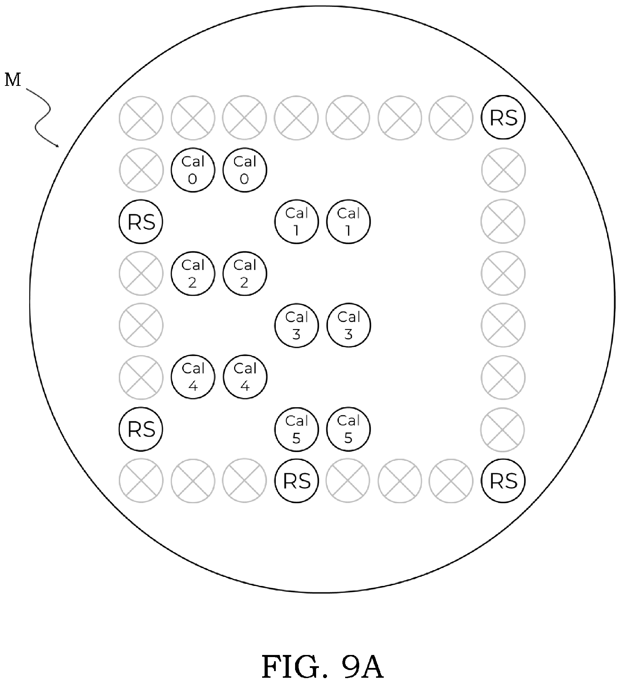

Figures 9A-9D show examples of calibration layouts. Specifically, as mentioned above, the calibration may be organized as follows:

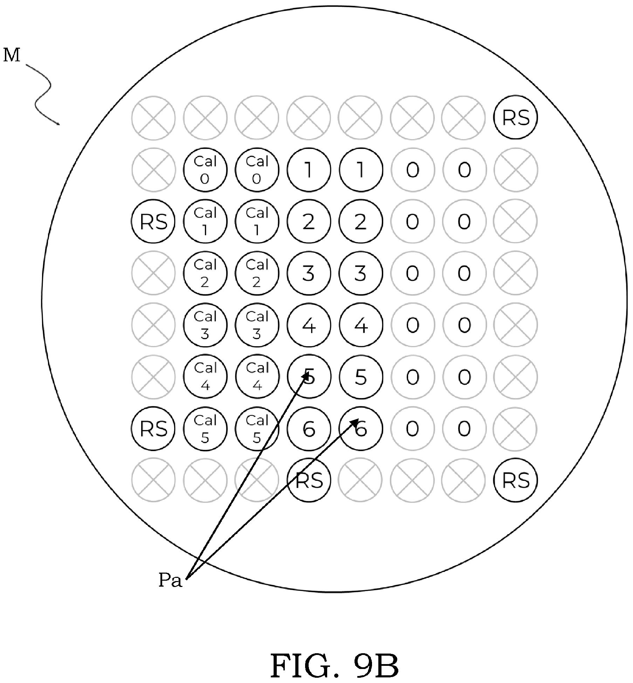

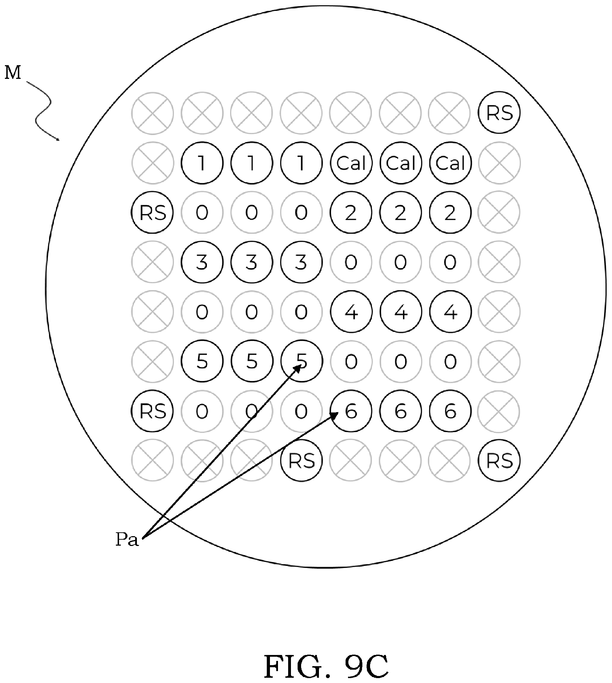

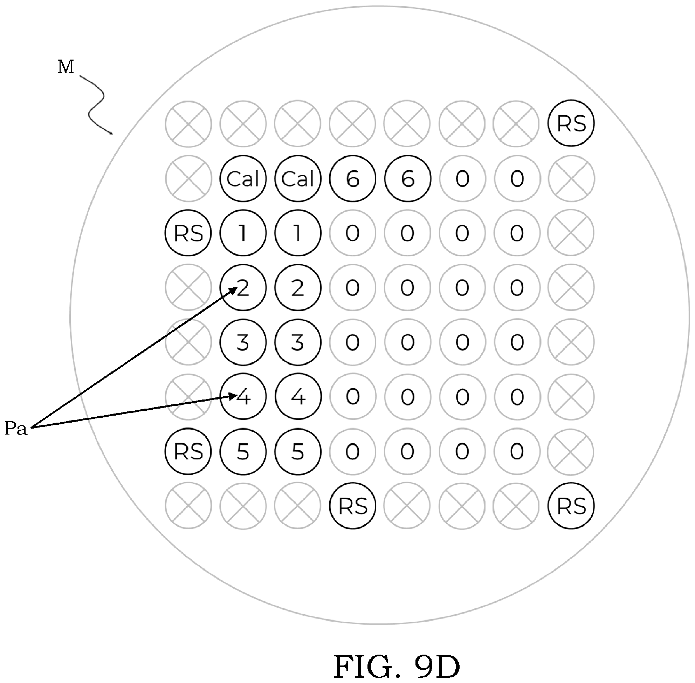

- a dedicated well inserted in a separate monotest calibration device, which is aimed at calibrating the specific batch in a certain apparatus, or a dedicated well inserted in each monotest device (in addition to the analysis well), which is aimed at performing a calibration for each tested sample (see the example of Figure 9A), may be used;
- in addition or alternatively, calibration points inserted together with the analysis parameters inside a single well may be provided, thus allowing a calibration for each tested sample in the same solid phase (see the example of Figure 9B);
- finally, in addition or alternatively, should the test be more suitable with a qualitative solution, a single calibration point with such an intensity as to allow the calculation of an intensity index for each parameter involved is provided (see the examples of Figures 9C and 9D).

The above-mentioned calibration may be used, for example, to define the above-mentioned intensity index to be assigned to each of the parameters. In particular, once the intensity of a parameter has been calculated (i.e. as the average of the spots relative to said parameter in the above-indicated manner), the intensity index for each parameter is defined as the ratio between the calculated intensity and a calibration factor. Thus, the calibration allows to determine relative units for each single parameter in the panel, or an index for the purpose of defining a qualitative result (positive, negative or equivocal).

This calculation thus allows to provide the final result, wherein, for example, a sample is considered positive if said index is higher than 1.1, negative if said index is lower than 0.9, whereas the result is dubious if it has a value between 0.9 and 1.1.

As shown in the figures, there are many possible layouts for the matrix M, and the present invention is not limited to a particular layout, wherein the most suitable layout may be selected based on the specific applications.

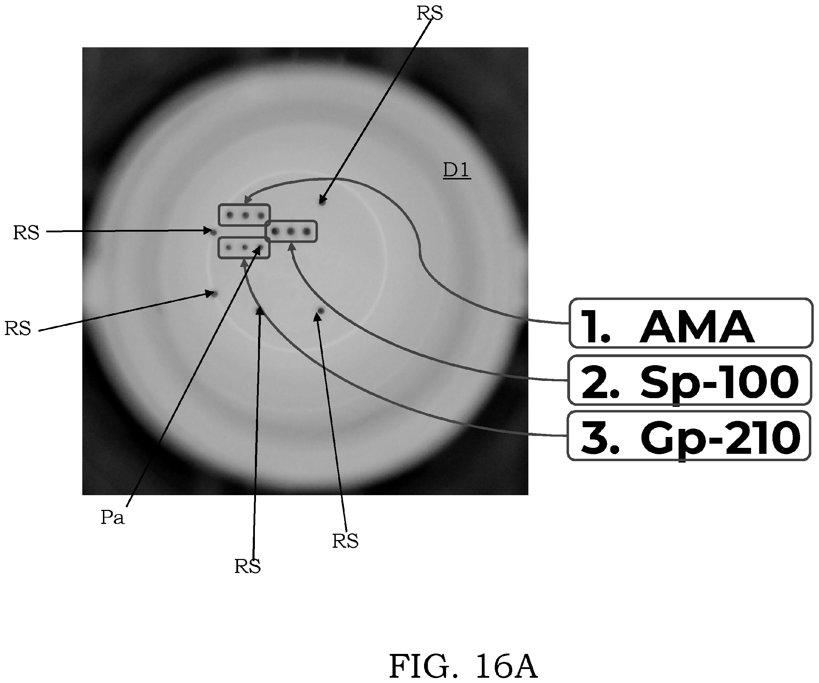

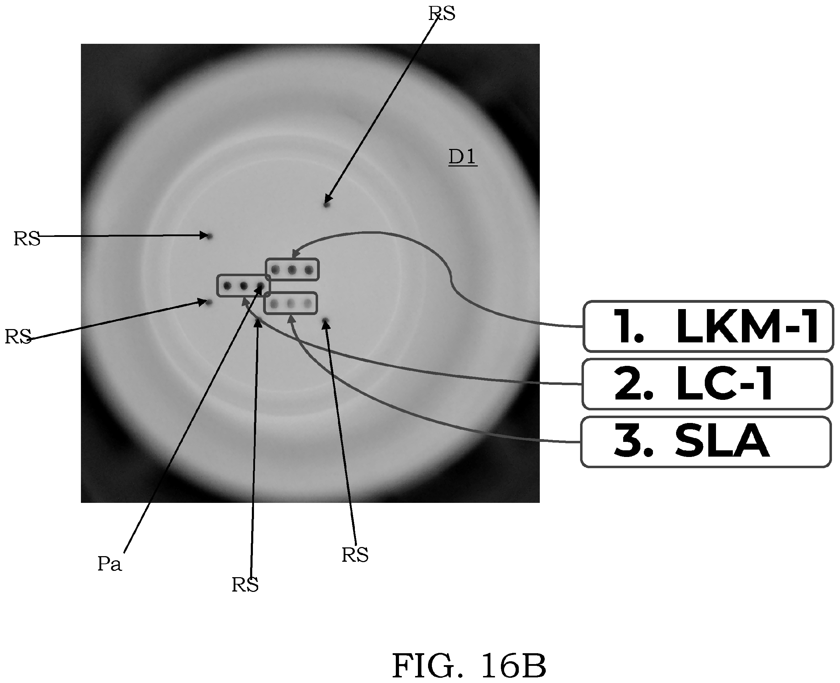

Finally, with reference to Figure 16A and 16B, a preferred example of application of the apparatus 1 of the present invention is described below, in particular an innovative diagnostic macroarray test for autoimmune diseases, for example autoimmune hepatopathy (EAI) and primary biliary cirrhosis (PBC), is described.

Autoimmune hepatopathy is in particular a disease characterized by chronic liver inflammation, caused by an autoimmune process, and affects especially women of age between 40 and 70 years.

Depending on the type of autoantibodies produced by the organism, there are mainly two forms of autoimmune hepatitis, known as type-1 autoimmune hepatitis (EAI-1) and type-2 autoimmune hepatitis (EAI-2).

The autoantibodies that are typically the EAI-1 biomarkers comprise: the antinuclear autoantibody (ANA), the anti-smooth muscle autoantibody (ASMA) and the anti-soluble liver antigen autoantibody (anti-SLA). The latter, in particular, may be used as single biological marker for EAI-1 diagnosis.

The autoantibodies that are typically the EAI-2 biomarkers comprise: the anti-liver-kidney microsomal autoantibody (anti-LKM1), and the anti-liver cytosolic antigen type-1 autoantibody (anti-LC1).

Primary biliary cirrhosis (PBC) is a chronic autoimmune hepatopathy, characterized by the progressive destruction of the bile ducts that pass inside the liver, and it occurs almost exclusively in women of age between 35 and 70 years.

The antibodies that are typically the PBC biomarkers comprise: the anti-mitochondrial autoantibody (AMA-M2, wherein M2 is a specific protein part of the mitochondrion against which the AMAs are directed), the anti-F-actin antibody (anti-F-actin), the sp100 antibody (anti-sp100) and the gp210 antibody (anti-gp210).

It is noted that, within the scope of the present invention and as it is known in the art, the terms "biomarker" and "analyte" are herein used interchangeably, and refer to a biological indicator, such as for example a protein, that is related to a specific disease.

Referring in particular to Figure 16A, such figure shows an example of reactivity, tested in triplicate, of a sample at the points of analysis Pa of matrix M of the reaction well D1 that are intended for the biomarkers AMA, anti-sp100 and anti-gp210, that are typical of primary biliary cirrhosis.

Referring in particular to Figure 16B, such figure shows an example of reactivity, tested in triplicate, of a sample at the points of analysis Pa of matrix M of the reaction well D1 that are intended for the biomarkers anti-LKM-1, anti-LC-1 (that are typical of EAI-2) and anti-SLA (that is typical of EAI-1).

Advantageously, the apparatus according to the present invention allows the detection and the determination of at least one biomarker, preferably two biomarkers, more preferably at least three biomarkers, even more preferably at least four biomarkers, conveniently at least five biomarkers, by means of a single macroarray test.

In a particularly preferred embodiment, the present invention allows the detection of six biomarkers, by means of a single macroarray test, said biomarkers preferably being AMA, anti-sp100, anti-gp210, anti-LKM-1, anti-LC-1 and anti-SLA.

Positivity and the value of the sample (titer) are defined on the basis of a cut off that has been determined experimentally, based on the titer, obtained during experimentation, on positive and negative known samples. The value of the sample is then given in Index (ratio of the signal obtained on the sample/cut-off signal), or in AU/mL (AU= Arbitrary Unit); in the latter case, the signal of the sample is interpolated on a known curve, to which arbitrary units are assigned. Also in the case in which the value of the sample is defined using AU/mL, the positivity threshold is determined experimentally by using positive and negative known samples.

In conclusion, the apparatus according to the invention achieves the set goals and has numerous advantages which have been already partially mentioned, thus brilliantly overcoming the technical problem and solving all the drawbacks of the prior art.

Advantageously according to the present invention, macroarray tests are performed on devices which are of the single use-type for a single determination, thus obtaining an effective and at the same time very simplified apparatus that is capable of rapidly performing said tests.

The macroarray technology allows to perform multiple tests on a single device (which is useful, for example, in the allergy and autoimmunity fields), and, by means of the apparatus of the present invention, it is possible to perform all the tests of interest by means of monotest kits (i.e. the above-mentioned test devices for a single determination) that are already available and used also on other instruments.

For example, the capture-aimed antigens in solid phase and the reference spots are purified native proteins or recombinant proteins, that are printed on the bottom of 96-well microplates, arranged in a matrix that is similar to the previously shown alternatives; the wells are then divided into single units and inserted into the above-described analysis device.

The single-use devices carry aboard all the reagents required for the test and a reaction well for the simultaneous determination of the selected biomarkers in a single macroarray test, for example the above-mentioned six biomarkers for type-1 autoimmune hepatopathy (EAI-1), type-2 autoimmune hepatopathy (EAI-2) and primary biliary cirrhosis (PBC), but possibly also for the detection and the determination of biomarkers related to other autoimmune diseases.

In a preferred example, the present invention allows the clinician to obtain multi-parameter information regarding the above-mentioned biomarker autoantibodies related to EAI-1, EAI-2 and PBC. The present invention also allows to work on monotest devices with rapid response times, and allows the use of a minimum volume of sample for numerous simultaneous determinations (for example, six simultaneous determinations).

As illustrated, by means of the above-mentioned apparatus, it is further possible to perform even simultaneously, various types of tests in addition to the macroarray, such as ELISA test, CLIA test.

A further big advantage consists in the possibility of "continuous" loading the test devices, which allows to continuously insert more samples and reagents, even in the case in which an immediate analysis of the same is not possible. The user has only the task of positioning the test tubes and the test devices with the reagents into the appropriate housings (possibly paying attention to expose the respective barcode in the side covered by the reader), the system then providing for everything else. The functionality of continuous loading is always available; this guarantees, in addition to the above-mentioned versatility, also a great flexibility. In fact, the addition of the outer pre-cycle rotor, under ordinary working conditions, allows the user to immediately load possible samples to be analyzed at a later time even if they are incompatible with the ongoing analyses. In this way, the temperature and vibration incompatibility of some samples is limited, if not totally solved. Advantageously, the user may then interact with the apparatus when (s)he desires to introduce new tests (provided that there are the required spaces in the racks or in the pre-cycle rotor and that the samples in the racks are not in the step of transfer); it is not necessary to wait the end of the ongoing analyses to be able to load new samples / devices.

In any case, in general, the main advantage is the possibility to perform macroarray tests on monotest devices with rapid response times.

Obviously, a person skilled in the art, in order to meet particular needs and specifications, may carry out several changes and modifications to the apparatus described above, all included in the protection scope of the invention as defined by the following claims.

The present invention will be further described with reference to some exemplifying embodiments which are provided below by way of non-limiting illustration.

Example 1 - Detection of AMA, anti-sp100 and anti-gp210 in a single macroarray test.

Figure 16A shows an image obtained on a single solid phase of a well, in which the detection of the below-mentioned biomarkers has occurred, thereby proving that, from a sample of serum coming from a patient suspected of suffering from autoimmune hepatopathies and containing a set of different biomarkers, it is possible a multi-parameter medical reporting which is useful for diagnosis.

Materials:

M2 antigens: recombinant proteins PDC-E2, OGDC-E2, BCOADC-E2;

Sp100: recombinant protein Sp100;

gp210: recombinant protein of multiple connected or non-connected copies of short cytoplasmic C-terminal domains gp-210;

(provided in this example by the firm "BBI Solutions").

The protein molecules are diluted to the concentration of use, for the subsequent dispensing by means of "non-contact printer", in buffer solutions selected on the basis of the special chemical-physical features of the single recombinant proteins. In particular, phosphate buffer (concentration 0.01M), TRIS buffer (concentration 0.07M), and carbonate buffer (concentration 0.1M) was used, with pH adjustment based on the single antigen.

Binding surface

The set of recombinant proteins, based on their average amino acid composition, was dispensed on a hydrophilic surface made of high-affinity (HB) polystyrene - in this example provided by the firm Biomat - recommended for passive adsorption of proteins with various hydrophilicity levels.
The binding surface is organized in a "breakable" 96-well format, to allow the division of the plates into single wells intended for the single-use device, white-colored to provide a non-transparent surface for the detection of the colored precipitate by means of acquisition of high-resolution images.

Methods:

The immunoenzymatic method according to the present invention allows to identify the above-mentioned autoantibodies, recognized by the scientific community as biomarkers characterizing a distinct profile of autoimmune hepatopathy in PBC and EAI type-1 and type-2
In a serum sample of a human patient, it is possible to identify said autoantibodies by allowing them to bind the related epitope, which is immobilized in solid phase, and thus to identify the bound analyte by means of the subsequent formation of an immunocomplex with a secondary antibody that in turn conjugates an enzyme catalyzing the oxidation of a specific substrate with the formation of a permanent colored deposit.
The method according to the present invention was performed according to the following steps:

1) sensitization of the binding surface with recombinant (or, if necessary, native) proteins by means of "non-contact printer" (i.e. Nano-Plotter 2.1, GeSiM) provided with piezoelectric tips that allow punctual and contactless dispensing of about 200 p1 of antigen solution on a matrix organized in a predefined multi-parameter layout; followed by a 1-hour incubation at 37°C, washing with aqueous solution (i.e.

H₂O, Brij 0.005%), and treatment of the well with a saturating agent (i.e. PBS buffer solution pH 7.2-7.4, 6-7% saccharose and 0.5-1.0% BSA) for 1 hour; finally, drying of the solid phase;

2) Incubation of the sample for 15' at RT, diluted in a suitable diluent of the samples (1:100 in PBS Buffer);

3) Washing with a solution of phosphate buffer and detergent to remove everything that has not bound;

4) Incubation of the conjugate (secondary tracer) for 15' at RT; the tracer used was an anti-human IgG antibody conjugated with horseradish peroxidase (HRP) diluted in PBS buffer + 2% of fetal bovine serum;

5) Washing with a solution of phosphate buffer and detergent to remove everything that has not bound;

6) Incubation of the substrate for 15' at RT;

7) Detection of the possible precipitate formed in the predefined spots in the coating matrix, by means of a camera and an analysis software.

Example 2 - Detection of anti-LKM-1, anti-LC-1, anti-SLA in a single macroarray test

For the detection of anti-LKM-1, anti-LC-1, anti-SLA in a single macroarray test, a macroarray test according to the materials and methods indicated in Example 1 was performed, using:

LKM-1: recombinant protein P450;

LC1: recombinant protein FTCD also known as LC1;

SLA/LP: recombinant protein SLA/LP;

(provided in this example by the firm "BBI Solutions").

Figure 16B shows an image obtained on a single solid phase of a well, in which the detection of the above-mentioned biomarkers has occurred.

**Claims**

**1.** An apparatus (1) for performing immunometric tests, comprising:

- a housing element (2) configured to receive and allow tests on at least one test device (D) which is of the single use-type for a single sample test, and which comprises at least one reaction well (D1) containing a solid phase, a plurality of wells (D2) for reagents, and at least one well (D3) for a sample to be analyzed;
- a control unit (C) apt to manage the apparatus (1), and
- at least one image detector (40) in communication with the control unit (C) for acquiring images (Img) of the reaction well (D1),

wherein the control unit (C) is configured to process the acquired images (Img) and to perform, on the basis of said processing, a macroarray test relative to the sample in said test device (D),
wherein, in said processing, the control unit is configured to identify, at the reaction well (D1) of said test device (D), points of a matrix (M), wherein said matrix (M) comprises points of analysis (Pa) related to at least one specific biomarker that represent measurement parameters of said macroarray test, and to perform said macroarray test on the basis of said points, and
wherein the test device (D) comprises a body (D') which houses, along its longitudinal axis (H-H), the reaction well (D1) containing a solid phase, the plurality of wells (D2) for reagents, and the well (D3) for the sample to be analyzed, and wherein said test device (D) comprises, along said longitudinal axis (H-H), at least one recess to receive the reaction well (D1) from a preexisting microtitration plate.

2. The apparatus (1) according to claim 1, wherein the matrix (M) also comprises a plurality of reference points (RS) in a fixed position, said reference points (RS) being arranged to allow to recognize the orientation of the points of analysis (Pa) of the matrix (M) within the acquired image (Img), and wherein the control unit (C) is configured to perform a procedure of virtual alignment of matrix (M) on the basis of said reference points (RS).

3. The apparatus (1) according to claims 1 or 2, wherein the control unit (C) is configured to process the acquired images (Img) by means of machine learning techniques.

4. The apparatus (1) according to any one of the preceding claims, wherein the control unit (C) is configured to identify and define the points of the matrix (M) by implementation of a calculation procedure based on Hough transform.

5. The apparatus (1) according to any one of the preceding claims, wherein the control unit (C) is configured to:

- define, for each image (Img) and for each of the identified points of analysis (Pa), a pixel array including the pixels included in said single points of analysis (Pa); and
- define also, for each image (Img), a pixel array including the pixels not included in said identified points of analysis (Pa).

6. The apparatus according to claim 5, wherein the control unit (C) is further configured to calculate the average value of the pixels in said pixel arrays and to calculate, on the basis of said average values, the intensity of each of the identified points of analysis (Pa).

7. The apparatus (1) according to any one of the preceding claims, wherein the control unit (C) is configured to perform a calibration procedure based on one or more known calibration points (Cal) within the matrix (M) that represent the dynamic range of the measurement parameters, said calibration points (Cal) being defined within a calibration well specifically apt to perform said calibration procedure or within the matrix of the reaction well (D1) itself.

8. The apparatus (1) according to any one of the preceding claims, wherein the matrix (M) comprises at least one 6x6 grid or one 8x8 grid for the points of analysis (Pa), and wherein each measurement parameter is tested in duplicate or triplicate within the reaction well (D1).

9. The apparatus (1) according to any one of the preceding claims, wherein the solid phase comprises purified native proteins or recombinant proteins that are printed on the bottom of said microtitration plate, which is subdivided into said reaction wells (D1) which are individually inserted into said test device (D), and wherein the matrix (M) is formed by means of a coating of said reaction well (D1).

10. The apparatus (1) according to any one of the preceding claims, comprising one or more needles controlled in an automatic way by the control unit (C) for transferring the samples and reagents for carrying out the immunometric test, and wherein said control unit (C) is configured to control said needles so as to perform the following analysis steps:

- addition of the sample to be analyzed into the reaction well (D1) and incubation of the same in said reaction well (D1);
- washing of the reaction well (D1);
- addition of a conjugated tracer into the reaction well (D1) and incubation of the same in said reaction well (D1);
- further washing of the reaction well (D1); and
- addition of a macroarray substrate into the reaction well (D1).

11. The apparatus (1) according to any one of the preceding claims, wherein the housing element (2) is configured to house a plurality of said test devices (D), said apparatus (1) further comprising means (4) apt to act on the housing element (2) to allow the execution of the immunometric test.

12. The apparatus (1) according to any one of the preceding claims, wherein said biomarkers are related to an autoimmune disease.

13. The apparatus (1) according to claim 12, wherein said biomarkers comprise the antinuclear autoantibody (ANA), the anti-smooth muscle autoantibody (ASMA) and the anti-soluble liver antigen autoantibody (anti-SLA), said autoimmune disease being type-1 autoimmune hepatopathy (EAI-1).

14. The apparatus (1) according to claim 12, wherein said biomarkers comprise: the anti-liver-kidney microsomal autoantibody (anti-LKM1), and the anti-liver cytosolic antigen type-1 autoantibody (anti-LC1), said autoimmune disease being type-2 autoimmune hepatopathy (EAI-2).

15. The apparatus (1) according to claim 12, wherein said biomarkers comprise: the anti-mitochondrial autoantibody (AMA), the anti-M2 autoantibody (anti-M2), the anti-F-actin antibody (anti-F-actin), the sp100 antibody (anti-sp100) and the gp210 antibody (anti-gp210), said autoimmune disease being primary biliary cirrhosis (PBC).

16. The apparatus (1) according to any one of the preceding claims, wherein said matrix (M) includes points of analysis (Pa) related to at least two biomarkers, preferably at least three biomarkers, more preferably at least four biomarkers, even more preferably at least five biomarkers, even more preferably six biomarkers selected from biomarkers of EAI-1, EAI-2 and/or PBC, said six biomarkers preferably being the anti-mitochondrial autoantibody (AMA), the sp100 antibody (anti-sp100), the gp210 antibody (anti-gp210), the anti-liver-kidney microsomal autoantibody (anti-LKM1), the anti-liver cytosolic antigen type-1 autoantibody (anti-LC1), and the anti-soluble liver antigen autoantibody (anti-SLA).

17. A method for performing immunometric tests, comprising:

- arranging a housing element configured to receive and allow tests on at least one test device which is of the single use-type for a single sample test, and which includes at least one reaction well containing a solid phase, a plurality of wells for reagents, and at least one well for a sample to be analyzed;
- acquiring images of the reaction well;
- processing the images acquired, wherein said processing comprises identifying, at the reaction well of said test device, points of a matrix, wherein said matrix comprises points of analysis related to at least one specific biomarker that represent measurement parameters of said macroarray test; and
- performing, on the basis of said processing, a macroarray test relative to the sample in the test device.

18. The method according to claim 17, wherein the matrix comprises at least one 6x6 grid or one 8x8 grid for the points of analysis, and wherein each measurement parameter is tested in duplicate or triplicate within the reaction well.

19. The method according to claim 17, wherein said biomarkers are related to an autoimmune disease.

20. The method according to claim 19, comprising the step of selecting the biomarkers from: the antinuclear autoantibody (ANA), the anti-smooth muscle autoantibody (ASMA) and the anti-soluble liver antigen autoantibody (anti-SLA), said autoimmune disease being type-1 autoimmune hepatopathy (EAI-1).

21. The method according to claim 19, comprising the step of selecting the biomarkers from: the anti-liver-kidney microsomal autoantibody (anti-LKM1), and the anti-liver cytosolic antigen type-1 autoantibody (anti-LC1), said autoimmune disease being type-2 autoimmune hepatopathy (EAI-2).

22. The method according to claim 19, comprising the step of selecting the biomarkers from: the anti-mitochondrial autoantibody (AMA), the anti-M2 autoantibody (anti-M2), the anti-F-actin antibody (anti-F-actin), the sp100 antibody (anti-sp100) and the gp210 antibody (anti-gp210), said autoimmune disease being primary biliary cirrhosis (PBC).

23. The method according to claim 17, wherein the matrix includes points of analysis related to at least two biomarkers, preferably at least three biomarkers, more preferably at least four biomarkers, even more preferably at least five biomarkers, even more preferably six biomarkers selected from biomarkers of EAI-1, EAI-2 and/or PBC, said six biomarkers preferably being the anti-mitochondrial autoantibody (AMA), the sp100 antibody (anti-sp100), the gp210 antibody (anti-gp210), the anti-liver-kidney microsomal autoantibody (anti-LKM1), the anti-liver cytosolic antigen type-1 autoantibody (anti-LC1), and the anti-soluble liver antigen autoantibody (anti-SLA).

24. Use of the apparatus (1) according to any one of the claims from 1 to 11 in the detection of autoimmune diseases.

FIG. 1

FIG. 2

H

D'

D3

D2

D1

D4

D

H

FIG. 3

FIG. 4A

FIG. 4B

FIG. 5

FIG. 6

FIG. 7

EP 4 782 841 A2

FIG. 8

FIG. 9A

FIG. 9B

FIG. 9C

EP 4 782 841 A2

FIG. 9D

FIG. 10

EP 4 782 841 A2

FIG. 11

EP 4 782 841 A2

FIG. 12

EP 4 782 841 A2

EP 4 782 841 A2

**Fp**

30, 40

10

8

20

2s

FIG. 13

FIG. 14

EP 4 782 841 A2

FIG. 15

FIG. 16A

FIG. 16B